Europäisches Patentamt

(19) European Patent Office

Office européen des brevets

(11) Numéro de publication: **0 284 588**
**B1**

(12) **FASCICULE DE BREVET EUROPEEN**

(45) Date de publication du fascicule du brevet: **16.01.91**

(21) Numéro de dépôt: **88870036.6**

(22) Date de dépôt: **09.03.88**

(51) Int. Cl.⁵: **A 61 K 31/405,** A 61 K 47/08, A 61 K 47/22

(54) **Préparation pharmaceutique à base d'indométacine.**

(30) Priorité: **13.03.87 BE 8700257**

(43) Date de publication de la demande: **28.09.88 Bulletin 88/39**

(45) Mention de la délivrance du brevet: **16.01.91 Bulletin 91/03**

(84) Etats contractants désignés: **BE CH DE FR GB IT LI NL SE**

(56) Documents cités:
**US-A-4 082 881**

**CHEMICAL ABSTRACTS, vol. 105, no. 20, 17 novembre 1986, page 381, résumé no. 178434u, Columbus, Ohio, US; & FI-A-69 565 (OSAKEYTHIO STAR AB) 29-11-1985**

**PATENT ABSTRACTS OF JAPAN, vol. 7, no. 232 (C-190)1377r, 14 octobre 1983; & JP-A-58 124 716 (SUMITOMO KAGAKU KOGYO K.K.) 25-07-1983**

(73) Titulaire: **"PHARLYSE", Société Anonyme Boulevard Royal, 2 Luxembourg (LU)**

(72) Inventeur: **Baudier, Philippe Raymond Avenue Blücher, 10 B-1410 Waterloo (BE)**
Inventeur: **Fossion, Jacques Jean Rue du Cours d'Eau, 18 B-1428 Braine- L'Alleud (BE)**
Inventeur: **De Boeck, Arthur Marie Barrio Navarro Box 705 Gurabo Puerto Rico 00658 (US)**
Inventeur: **Maes, Paul José Rue Porte de Lorette, 76 B-4540 Vise (BE)**

(74) Mandataire: **Gaspar, Florent et al Bureau VANDER HAEGHEN 63, avenue de la Toison d'Or B-1060 Bruxelles (BE)**

Courier Press, Leamington Spa, England.

**Description**

La présente invention est relative à une préparation pharmaceutique liquide à usage topique cutané à base d'indométacine comme ingrédient actif.

L'invention permet d'administrer localement par voie externe et de façon précise et aisée l'indométacine sous forme de solution.

De tous les organes du corps humain, la peau est le plus accessible et le plus étendu. D'une épaisseur de quelques dixièmes de millimètre, elle se comporte comme une barrière protectrice très efficace vis-à-vis des attaques physiques et des agents chimiques. Depuis des siècles, de nombreuses tentatives de traitement par voie topique des maladies du corps ont été faites, mais ce n'est que depuis ces dernières décades que quelques résultats positifs ont pu être obtenus.

En particulier, dans le domaine des maladies inflammatoires, de nombreux traitements par voie transcutanée ont été tentés, comme par exemple au moyen de produits contenant des rubéfiants. Ces derniers procurent une sensation superficielle de chaleur par un phénomène de vasodilatation locale, mais ont peu ou pas d'activité sur la réduction de l'inflammation ou de la douleur. Plus récemment, des formes topiques à base soit de corticoïdes, soit d'anti-inflammatoires non stéroïdiens sont apparues sur le marché.

L'intérêt du traitement local au moyen d'anti-inflammatoires non stéroïdiens, dans les affections inflammatoires musculo-squelettiques, s'avère très grand car la douleur siège, dans la majorité des cas, dans des zones limitées. Ce genre de traitement possède l'avantage psychologique évident de traiter la zone douloureuse elle-même et il permet, en outre, d'éviter la voie orale et donc de réduire voire d'éviter complètement, les effets secondaires systémiques importants et fréquemment liés à ce type de médicament, tels que les troubles gastro-intestinaux par exemple.

L'indométacine ou acide 1-(p-chlorobenzoyle)-5-méthoxy-2-méthylindole-3-acétique est une substance bien connue en thérapeutique, particulièrement pour ses remarquables propriétés anti-inflammatoires et analgésiques. Dans la classe des anti-inflammatoires non stéroïdiens, elle constitue un des produits les plus actifs et les plus étudiés, ce qui en fait un médicament de premier choix dans le traitement de ces maladies inflammatoires.

Elle présente néanmoins l'inconvénient lorsqu'elle est administrée par la voie buccale, d'entraîner fréquemment des effets secondaires importants, tels que des irritations des muqueuses digestives pouvant aboutir à des gastrites simples ou hémorragiques et même à des ulcères gastro-intestinaux.

Ces inconvénients permettent de comprendre aisément l'importance qu'il y a à trouver d'autres voies d'administration permettant d'éviter la présence d'indométacine dans le tractus gastro-intestinal.

Une forme à usage topique cutané devra présenter non seulement d'excellentes propriétés de pénétration du médicament au niveau de la zone douloureuse, mais encore une absence de réaction cutanée d'intolérance et en outre permettre une application aisée.

Actuellement, il existe, à la disposition du corps médical, une préparation topique d'indométacine sous forme d'onguent ou de gel. Ce type de préparation, bien que possédant une activité thérapeutique certaine, présente toutefois le désavantage, d'une part, d'un manque de précision dans la quantité appliquée et, d'autre part, de rendre souvent souhaitable le recouvrement, au moyen d'un pansement, de la zone d'application, afin de ne pas tacher les vêtements pouvant être mis en contact avec cette zone.

Une forme liquide pulsée, à base d'indométacine à usage topique, LUIFLEX®, a été introduite récemment sur le marché. Cette forme, bien que permettant de réaliser une application aisée et précise du médicament, tout en évitant au maximum les risques de souillure des vêtements, ne possède pas les qualités requises. En effet, ainsi qu'on le verra plus loin, cette forme non seulement manque d'efficacité mais, de plus, elle entraîne d'importantes intolérances cutanées.

La présente invention concerne une nouvelle préparation pharmaceutique, à base d'indométacine, destinée à être appliquée sur la peau à l'endroit de la douleur par pulvérisation ou par tout autre moyen adéquat.

Suivant la présente invention, la nouvelle préparation pharmaceutique à base d'indométacine est essentiellement caractérisée en ce qu'elle est constituée par une solution d'indométacine dans un mélange de diméthylisosorbide et d'isopropanol.

Le diméthylisosorbide est du 1,4:3,6-dianhydro-2,5-di-o-méthyl-D-glucitol (Atlas G—100, fabriqué par ICI, Grande Bretagne).

La nouvelle préparation suivant l'invention rend possible le passage de l'indométacine au travers de la peau jusqu'au siège même de l'inflammation, tout en n'entraînant pas de réactions cutanées secondaires d'intolérance.

Avantageusement, la préparation pharmaceutique suivant la présente invention est constituée d'une quantité d'indométacine comprise entre 0,5 g et 15 g, d'une quantité de diméthylisosorbide comprise entre 0,1 g et 60 g et de préférence de 1 g à 30 g et d'isopropanol en quantité suffisante pour faire 100 g.

La nouvelle préparation sous forme de solution peut être contenue dans un distributeur sous forme de nébulisateur muni d'une valve doseuse délivrant chaque fois une quantité connue et précise de la solution.

Avantageusement, la valve doseuse est du type pompe auto-amorçante, ce qui permet, comme dit plus haut, de rendre précise la quantité de liquide pulvérisé et ce, en évitant l'emploi d'un quelconque gaz propulseur. La quantité de liquide délivrée par la valve doseuse peut varier de 10 à 300 microlitres.

On donne ci-après des exemples de préparation et de conditionnement de la nouvelle forme galénique suivant l'invention.

### Exemple 1

| Indométacine | 5 g |
|---|---|
| Diméthylisosorbide | 18,5 g |
| Isopropanol q.s.p. | 100 g |

Après avoir introduit la quantité d'indométacine dans le diméthylisosorbide et la moitié de la quantité d'isopropanol, on chauffe légèrement le mélange, sous agitation, jusqu'à l'obtention d'une solution limpide. On ajoute alors le solde de l'isopropanol et la solution est rendue homogène.

### Exemple 2

| Indométacine | 5 g |
|---|---|
| Diméthylisosorbide | 12,5 g |
| Ether stéarylique de polyoxypropylène 15 | 12,5 g |
| Isopropanol q.s.p. | 100 g |

### EXEMPLE DE CONDITIONNEMENT

La solution préparée selon l'exemple 1 ou 2 est conditionnée par fractions de 25 ml dans des flacons en aluminium. Les flacons sont fermés par sertissage sur leur col d'une bague métallique munie d'une valve doseuse délivrant une quantité prédéterminée de solution à chaque pression sur un bouton poussoir. La préparation pharmaceutique pulsée, ainsi obtenue, permet de délivrer une quantité connue et précise d'indométacine sur la surface cutanée à traiter.

### A. Etude de l'activité anti-oedémateuse

Le test pharmacodynamique suivant permet de rendre compte, de façon objective, de l'efficacité des préparations pharmaceutiques étudiées.

L'étude est réalisée chez le rat en utilisant le test bien connu de l'oedème à la carragénine.

Une demi-heure avant l'induction de la réaction inflammatoire, la patte postérieure droite des animaux est plongée pendant une minute dans la solution à tester. La réaction inflammatoire est déclenchée par injection, au niveau sous-aponévrotique de la voûte plantaire, de 0,1 ml de solution physiologique contenant 1 % de carragénine.

| Influence des différents traitements sur le développement de l'oedème à la carragénine (n = 6) | |
|---|---|
| Produit testé | % d'augmentation du volume de la patte (moyenne) |
| Témoin | 54 ± 2 |
| Placebo (excipients seuls) | 47 ± 3 |
| Exemple 1 | 33 ± 3 |
| Exemple 2 | 37 ± 2 |
| Produit du marché* | 40 ± 3 |

\* Produit actuellement sur le marché : LUIFLEX®, composé d'indométacine 1 g, de myristate d'isopropyle, de parfum et d'isopropanol q.s.p. 100 g.

Les résultats de cette expérimentation montrent, à l'évidence, que l'indométacine administrée localement exerce un effet protecteur vis-à-vis d'une réaction inflammatoire aiguë.

La formulation contenant le diméthylisosorbide de l'exemple 1, objet de la présente invention, a procuré la plus grande protection vis-à-vis de l'oedème.

Une différence statistiquement significative [test t de STUDENT (voir SNEDECOR W. G. et COCHRAN G. W.: Statistical Methods (Iowa University Press, 1967, 6e éd.); SPIEGEL M. R.: Theory and Problems of Statistics (Schaum's collection, N.Y.))] au niveau $p < 0,01$ a été trouvée entre la formulation de l'exemple 1 et le produit actuellement sur le marché.

Ceci permet d'affirmer que la préparation pharmaceutique de l'exemple 1, objet de la présente invention, possède une activité thérapeutique nettement supérieure à celle de la préparation existant actuellement sur le marché.

B. Etude de la tolérance cutanée

La méthode utilisée a été la "Méthode officielle pour l'appréciation de l'agressivité superficielle cutanée par applications itératives" (J. O. de la République Française du 21 avril 1976).

Cette méthode est utilisée pour déterminer l'action sur la peau d'une application quotidienne d'un produit pendant plusieurs jours. On utilise des lapins albinos et les produits à tester sont appliqués sur le dos et le flanc préalablement tondus à la tondeuse électrique; une petite surface de l'arrièretrain, également tondue, ne reçoit pas de produit et sert de témoin.

On note régulièrement l'absence ou la présence d'érythème et d'oedème que l'on cote respectivement selon des échelles allant de 0 à 4 (0 = absence d'érythème ou d'oedème, 4 = érythème grave ou oedème grave). Lors de chaque observation, on calcule l'indice d'agressivité en additionnant les cotes des deux échelles et en divisant le résultat par le nombre total d'évaluations des phénomènes d'intolérance. La signification des indices obtenus s'établit comme suit:

Non irritant: indice inférieur ou égal à 0,5;

Légèrement irritant: indice compris entre 0,5 et 2;

Irritant: indice compris entre 2 et 5 et

Très irritant: indice compris entre 5 et 8.

N.B.: Mathématiquement, l'indice obtenu ne peut être supérieur à 8.

Les résultats, calculés après un mois d'applications quotidiennes, s'établissent comme suit:

| Résultats des tests de tolérance cutanée | | |
|---|---|---|
| Préparation testée | Indices après un mois | Signification |
| Placebo | 0 | non irritant |
| Exemple 1 | 0 | non irritant |
| Produit sur le marché* | 2,4 | irritant |

\* LUIFLEX®

Les excipients contenus dans le produit sur le marché et dans le produit de l'exemple 1, objet de l'invention, diffèrent uniquement par le remplacement du myristate d'isopropyle par le diméthylisosorbide.

Le myristate d'isopropyle, utilisé dans la formule du produit LUIFLEX®, est connu et décrit comme une substance fréquemment utilisée dans les préparations destinées à être appliquées sur la peau grâce à son absence de propriétés irritantes et sensibilisatrices (British Pharmaceutical Codex 1973).

Il est donc très surprenant de constater que ce myristate d'isopropyle est la cause des propriétés irritantes décelées dans la préparation du commerce, puisque son remplacement par du diméthylisosorbide dans la formule de l'exemple 1 fait disparaître complètement ces propriétés irritantes.

Ce fait est d'autant plus inattendu que la formule de l'exemple 1 contient un pourcentage d'indométacine environ quatre fois plus élevé que la formule du produit sur le marché et que l'indométacine, de par son caractère acide, est elle-même susceptible de provoquer des irritations cutanées.

L'ensemble des résultats permet d'affirmer que la préparation de l'exemple 1, objet de la présente invention, possède, outre une activité supérieure, des propriétés inattendues et surprenantes de parfaite tolérance cutanée.

**Revendications**

1. Préparation pharmaceutique liquide à usage topique cutané à base d'indométacine comme ingrédient actif, caractérisée en ce qu'elle contient du diméthylisosorbide et de l'isopropanol comme excipients.

2. Préparation pharmaceutique suivant la revendication 1, caractérisée en ce que la concentration en indométacine est comprise entre 5 mg/g et 150 mg/g.

3. Préparation pharmaceutique suivant la revendication 2, caractérisée en ce que la concentration en indométacine est d'environ 50 mg/g.

4. Préparation pharmaceutique suivant l'une quelconque des revendications 1 à 3, caractérisée en ce que la concentration en diméthylisosorbide est comprise entre 1 mg/g et 600 mg/g, de préférence entre 10 mg/g et 300 mg/g.

**Patentansprüche**

1. Flüssige Arzneizubereitung mit Indomethacin zur örtlichen Hautanwendung, dadurch gekennzeichnet, dass sie Dimethylisosorbide und Isopropanol als Bindemitteln enthält.

2. Arzneizubereitung nach Anspruch 1, dadurch gekennzeichnet, dass die Indomethacin Konzentration zwischen 5 mg/g und 150 mg/g liegt.

3. Arzneizubereitung nach Anspruch 2, dadurch gekennzeichnet, dass die Indomethacin Konzentration etwa 50 mg/g ist.

4. Arzneizubereitung nach einen der Ansprüche 1 bis 3, dadurch gekennzeichnet, dass die Dimethylisosorbide Konzentration zwischen 1 mg/g und 600 mg/g, vorzugsweise zwischen 10 mg/g und 300 mg/g liegt.

**Claims**

1. Liquid pharmaceutical preparation for cutaneous topic use containing indomethacin as active ingredient, characterized in that it contains dimethylisosorbide and isopropanol as excipients.

2. Pharmaceutical preparation according to claim 1, characterized in that the indomethacin concentration is comprised between 5 mg/g and 150 mg/g.

3. Pharmaceutical preparation according to claim 2, characterized in that the indomethacin concentration is about 50 mg/g.

4. Pharmaceutical preparation according to anyone of claims 1 to 3, characterized in that the dimethylisosorbide concentration is comprised between 1 mg/g and 600 mg/g, preferably between 10 mg/g and 300 mg/g.